(19) Europäisches Patentamt
European Patent Office
Office européen des brevets

(11) Publication number: **0 001 920**
**B1**

(12) **EUROPEAN PATENT SPECIFICATION**

(45) Date of publication of patent specification: **25.05.83**

(51) Int. Cl.³: **C 07 D 519/04,**
A 61 K 31/435

(21) Application number: **78300580.4**

(22) Date of filing: **02.11.78**

(54) **Vincaleukoblastine derivatives, a process for their preparation, pharmaceutical formulations containing them and the derivatives for use as antimitotic agents.**

(30) Priority: **07.11.77 US 848837**

(43) Date of publication of application:
**16.05.79 Bulletin 79/10**

(45) Publication of the grant of the patent:
**25.05.83 Bulletin 83/21**

(84) Designated Contracting States:
**DE GB NL SE**

(56) References cited:
**Medicinal Chemistry, third Edition (Ed. A. Burger), Wiley-Interscience, p. 740-741, Noble et al., Ann. N.Y. Acad-Sci. 76, 882—894 (1958), Neuss et al., Tetrahydron Letters, 783—787 (1968).**

(73) Proprietor: **ELI LILLY AND COMPANY**
**307, East McCarty Street**
**Indianapolis Indiana 46285 (US)**

(72) Inventor: **Wright, Ian Glaisby**
**300, Meander Way**
**Greenwood Indiana 46142 (US)**
Inventor: **Neuss, Norbert**
**6461 Sunset Lane**
**Indianapolis Indiana 46260 (US)**
Inventor: **Thompson, Gerald Lee**
**925, West 58th Street**
**Indianapolis Indiana 46208 (US)**

(74) Representative: **Hudson, Christopher Mark et al,**
**European Patent Attorney Erl Wood Manor**
**Windlesham Surrey GU20 6PH (GB)**

**The file contains technical information submitted after the application was filed and not included in this specification**

EP 0 001 920 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Vincaleukoblastine derivatives, a process for their preparation, pharmaceutical formulations containing them and the derivatives for use as antimitotic agents.

The invention relates to novel 4-desacetoxy-4α-hydroxyindole-dihydroindoles.

The Vinca alkaloids, a group of dimeric indole-dihydroindoles, have achieved considerable prominence as marketed or experimental chemotherapeutic drugs for the treatment of susceptible carcinomas, sarcomas, and leukemias. These agents are used both alone and in combination with other oncolytic agents. As a class, the Vinca alkaloids include compounds obtainable from the leaves of *Vinca rosea,* derivatives produced by chemical modification thereof and more recently, dimeric alkaloids produced by coupling two "monomeric" indoles via a modified Polonovski reaction-see Langlois and Potier, *Tetrahedron Letters,* 1099 (1976), Potier, et al., *J.C.S. Chem. Comm.,* 670 (1975), Kutney et al., *Heterocycles,* 3, 205 (1975) and Atta-ur-Rahman, *Tetrahedron Letters,* 2351 (1976).

A majority of the known Vinca alkaloids can be represented by the following formula:

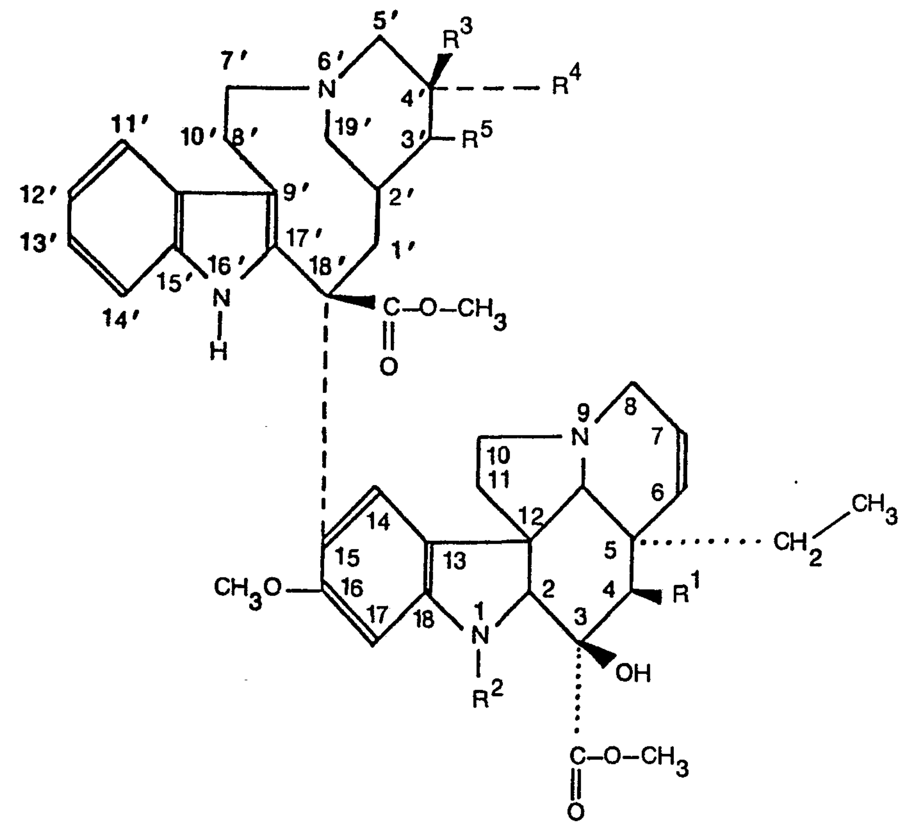

I

In the above formula, where $R^1$ is acetoxy, $R^2$ is methyl, $R^3$ is hydroxyl, $R^4$ is ethyl and $R^5$ is H, vinblastine is represented; where $R^1$ is acetoxy, $R^2$ is formyl, $R^3$ is hydroxyl, $R^4$ is ethyl and $R^5$ is H, vincristine is represented; where $R^1$ is acetoxy, $R^2$ is methyl, $R^3$ is ethyl, $R^4$ is hydroxyl and $R^5$ is H, leurosidine is represented; where $R^1$ is acetoxy, $R^2$ is methyl, $R^3$ and $R^5$ and H and $R^4$ is ethyl, deoxy VLB "A" is represented; where $R^1$, $R^2$ and $R^5$ are the same as in deoxy VLB "A" (4′-deoxyvinblastine) but $R^3$ is ethyl and $R^4$ is hydrogen, deoxy VLB "B" (4′-deoxyleurosidine) is represented; where $R^1$ is acetoxy, $R^2$ is methyl, $R^3$ is ethyl and $R^4$ and $R^5$ taken together form an α-epoxide ring, leurosine is represented; and where $R^1$, $R^3$, $R^4$ and $R^5$ are the same as in leurosine but $R^2$ is formyl, leuroformine (N-formylleurosine) is represented.

The above-mentioned alkaloids are described in the following publications: leurosine (vinleurosine — U.S. Patent No. 3,370,057), VLB (vincaleukoblastine, vinblastine — U.S. Patent No. 3,097,137), leurosidine (vinrosidine) and vincristine (leurocristine or VCR) (both in U.S. Patent No. 3,205,220), and deoxy VLB "A" and "B", *Tetrahedron Letters,* 783 (1958). Other alkaloids obtainable from vinca rosea include 4-desacetoxy vinblastine (U.S. patent 3,954,773); 4-desacetoxy-3′-hydroxyvinblastine (U.S. Patent 3,944,554); leurocolombine (2′-hydroxy VLB — U.S. Patent No. 3,890,325) and vincadioline (3′-hydroxy VLB — U.S. Patent 3,887,565).

Two of the above alkaloids, VLB and vincristine, are now marketed as drugs for the treatment of malignancies, particularly the leukemias and related diseases in humans. Of these marketed compounds, vincristine is a most active and useful agent in the treatment of leukemias but is also the least abundant of the antineoplastic alkaloids of *Vinca rosea.* Jovanovics et al. — U.S. Patent No. 3,899,493 — have developed an elegant oxidative procedure for converting the more abundant

alkaloid VLB to vincristine employing chromic acid in acetone and acetic acid at about −60°C. The same procedure has been used to prepare leuroformine (N-formylleurosine) from leurosine — see Belgian patent No. 811,110. Leuroformine is currently undergoing a clinical trial in Europe, chiefly in treatment of the leukemias and of multiple myeloma.

Chemical modification of VLB and vincristine has included hydrolysis of the 4-acetoxy group to yield 4-desacetyl VLB (DAVLB) or 4-desacetylvincristine (DAVCR) followed by reesterification with other acyl and amino-acyl groups — see U.S. Patents Nos. 3,392,173 and 3,387,001 — and replacement of the C—3 ester function by an amide function — see Belgian patent 837,390. One of the former 4-acyl derivatives, the 4-N,N-dimethylglycine ester underwent a brief clinical trial and one of the latter, vindesine, (4-desacetyl VLB C—3 carboxamide) is currently being tested clinically against a variety of neoplasms.

Other chemical modification of the VLB molecule such as hydrolysis and decarboxylation of the C—18′ carbomethoxy group has resulted in a loss of anti-cancer activity as has the formation of N-oxides; i.e., pleurosine (leurosine N-oxide). Oxidative attack on VLB under temperatures higher than −60°C. or in neutral or basic solution has resulted in the formation of a chemotherapeutically inactive compound, vinamidine, represented by the following formula:

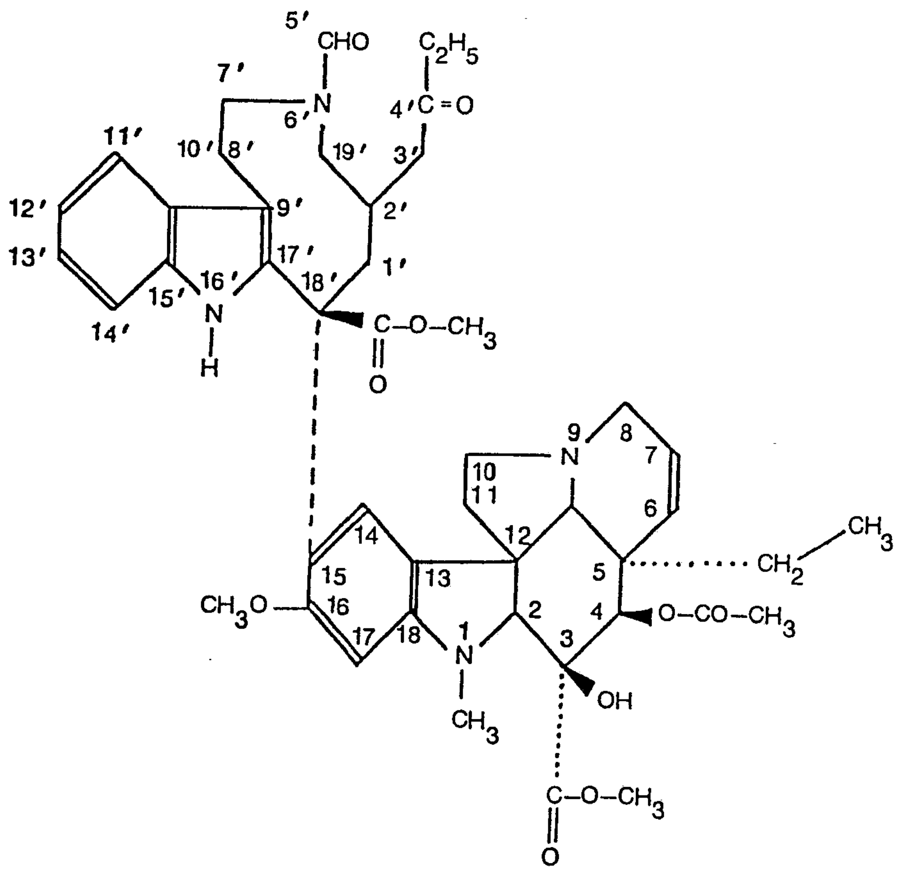

II

Oxidation of VLB with $MnO_2$ in acetone or $CH_2Cl_2$ at ambient temperature has also yielded vinamidine. The same alkaloid has been encountered in alkaloidal fractions from *Vinca rosea* leaves — see Tafur et al. *J. Pharm. Scio., 64,* 1953 (1975) — but the structure assigned therein (II on page 1956) is now believed to be incorrect and the above structure more closely represents the NMR, IR, and mass spectral data obtained from physiochemical studies of the compound. Vinamidine may arise from oxidative attack on VLB in which a vincinal 4′,5′-dihydroxy derivative is formed which glycol, upon further oxidation, splits between the hydroxyls-(4′,5′bond) to yield a ring-opened derivative such as II above.

4-Desacetyl VLB and other 4-desacetyl compounds; i.e., those in which $R^1$ in Formula I is hydroxyl, are not oxidized to the corresponding 4-oxo derivative by the Jovanovics low-temperature chromic acid oxidation (see Cullinan — Ser. No. 723,350). Theoretical considerations emphasize the difficulty of oxidizing the secondary hydroxyl at C—4 to a carbonyl. The ring containing the C—4 carbon is locked into position since four of the ring carbons are fused into other rings of the vindoline portion of the molecule. In order to undergo oxidation from a secondary hydroxyl to a carbonyl, the bond angles of the C—4 carbon would have to change from the tetrahedral angle (108°) to the $sp^2$

angle (120°). Such a change in the fused ring environment in which the C—4 carbon finds itself would involve considerable ring strain.

It is an object of this invention to prepare 4$\alpha$-derivatives of oncolytically active Vinca alkaloids having either a different anti-tumor spectrum, or lessened or different side effects, or both as compared with the marketed drugs, vinblastine and vincristine, and to provide the 4-oxo derivatives necessary for their preparation.

In fulfillment of the above and other objects, this invention provides an indole-dihydroindole of the formula

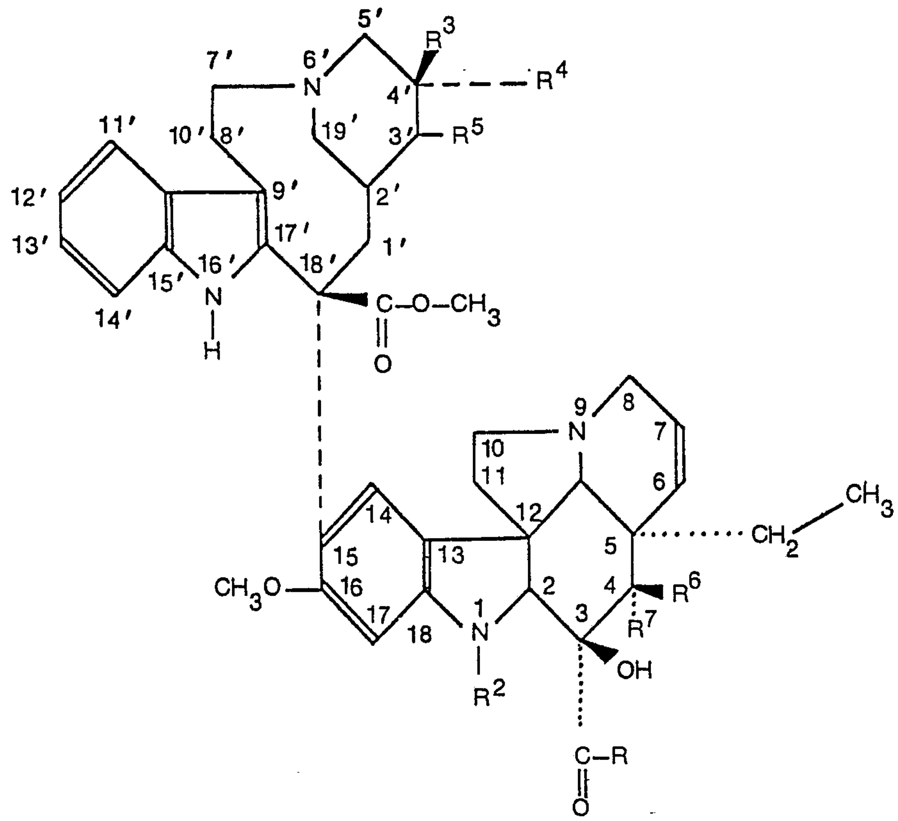

V

wherein:

$R^2$ is $CH_3$ or CHO;

when taken singly, $R^5$ is H and one of $R^3$ and $R^4$ is OH or H and the other is $C_2H_5$ and when $R^4$ and $R^5$ are taken together, they form an $\alpha$-epoxide ring and $R^3$ is $C_2H_5$, when taken singly $R^6$ is H and $R^7$ is $OR^1$ and when $R^6$ and $R^7$ are taken together they are =O;

$R^1$ is H or acetyl; and

R is $OCH_3$ or NH—$NH_2$ with the proviso that if R is —NH—$NH_2$, $R^1$ is H; and pharmaceutically acceptable acid addition salts thereof.

The invention also provide a process for preparing an indole-dihydroindole of the previously described formula V wherein:

$R^2$ is $CH_3$ or CHO;

when taken singly, $R^5$ is H and one of $R^3$ and $R^4$ is OH or H and the other is $C_2H_5$ and when $R^4$ and $R^5$ are taken together, they form an $\alpha$-epoxide ring and $R^3$ is $C_2H_5$,

$R^6$ is H;

$R^7$ is $OR^1$ wherein $R^1$ is H;

and R is $OCH_3$

which comprises reacting an indole-dihydroindole of the above formula wherein R, $R^1$, $R^2$, $R^3$, $R^4$ and $R^5$ are as above and wherein $R^6$ and $R^7$ together are =O with a reducing agent in a non-reactive anhydrous solvent and recovering as a free base or the pharmaceutically aceptable salts thereof; and optionally, preceded by reacting an indoledihydroindole of the above formula wherein R, $R^1$, $R^2$, $R^3$, $R^4$ and $R^5$ are as above and wherein $R^6$ is OH and $R^7$ is H with an oxidizing agent in a liquid phase selected from (a) N-chlorosuccinimide and dimethylsulfide with a tertiary ammine and (b) dicyclohexyl carbodiimide, o-phosphoric acid and dimethylsulfoxide (Moffat's Reagent).

The invention also provides a pharmaceutical composition which comprises an inert ingredient and as active ingredient an indoledihydroindole of the previously described formula V wherein $R^7$ is $OR^1$.

The invention also provides the use of the pharmaceutical composition as above described.

The compounds of formula V thus include, when $R^2$ is $CH_3$, 4-epi hydroxy or acetoxy (or

alternatively, 4$\alpha$-hydroxy or acetoxy) derivatives of vinblastine, leurosidine, leurosine, and deoxy VLB "A" and "B", all known oncolytic agents and when $R^2$ is CHO, derivatives of vincristine, 1-formylleurosine (leuroformine), 1-formylleurosidine, 4'-deoxy-1-formylleurosidine and 4'-deoxyvincristine, the latter two being disclosed in my copending US-application Serial No. 760,595 filed January 19, 1977, and also the 4-oxo derivatives which are intermediates in their preparation.

Also included within the scope of this invention are the pharmaceutically-acceptable acid addition salts of the above alkaloidal bases including salts derived from inorganic acids such as: hydrochloric acid, nitric acid, phosphoric acid, sulfuric acid, hydrobromic acid, hydriodic acid, nitrous acid, phosphorus acid and the like, as well as salts of organic acids including aliphatic mono and dicarboxylic acids, phenyl-substituted alkanoic acids, hydroxy alkanoic and alkandioic acids, aromatic acids, aliphatic and aromatic sulfonic acids. Such pharmaceutically-acceptable salts thus include the sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, 2-hydroxybutyrate, glycollate, malate, tartrate, methanesulfonate, propanesulfonate, naphthalene-1-sulfonate and naphthalene-2-sulfonate.

The 4-oxo indole-dihydroindole compounds of Formula V are prepared by oxidizing a 4$\beta$-hydroxy compound wherein $R_6$ is OH, $R_7$ is H and R is $OCH_3$, with Moffat's Reagent — dimethyl-sulfoxide (DMSO), dicyclohexylcarbodiimide (DCC) and ortho phosphoric acid. This reagent was originated by Pfitzner and Moffat, *J. Am. Chem. Soc., 85,* 3027 (1963) *87* 5661 (1965) — see also Fieser and Fieser, *Reagents for Organic Synthesis, 1,* 304, *2* 162 (John Wiley and Sons, Inc., 1967).

In carrying out the synthesis of the 4-oxo compounds (Formula III) of this invention with Moffat's reagent a 4$\beta$-hydroxy compound of Formula V, $R^6$ is OH (as the free base) and the dicyclohexylcarbodiimide are dissolved in DMSO at room temperature and the phosphoric acid is then added. The reaction mixture is agitated until solution is complete (5—10 minutes). The reaction flask is then stoppered and allowed to remain at room temperature until the reaction is substantially complete — 5 to 6 hours — as shown by TLC analysis of aliquot samples worked up by standard procedures. The desired product is isolated by diluting the reaction product with dilute sulfuric acid, filtering the resulting mixture and washing the precipitated dicyclohexylurea and 1 percent aqueous sulfuric acid. The filtrate is made basic with 14 N aqueous ammonium hydroxide and extracted into a water immiscible organic solvent such as methylene dichloride or ether. The organic extracts are combined, dried, and the solvent removed by evaporation. The residue containing the desired 4-oxo compound is purified by chromatography, gradient elution chromatography, or gradient pH extraction. The latter procedure is preferred.

In carrying out the above-mentioned oxidation employing Moffat's Reagent, it is customary to use an excess of the reagent. For example, an excess of dicyclohexylcarbodiimide of 2—10 fold and 2 to 5 fold excess of ortho phosphoric acid are employed. Dimethylsulfoxide is used as both a reactant and a solvent and it is therefore, always in a 10 to 20 fold excess. It is preferred to employ a 6—7 molar excess of dicyclohexylcarbodiimide and a 2.5 to 3 molar excess of ortho phosphoric acid per mole of starting material. The reaction can be carried out at temperatures ranging from 20°C. to 50°C., but is is preferred to employ temperature of about 20°C. As would be expected, the higher the temperature, the faster the reaction. However, temperatures in excess of about 25°C. may give rise to excessive quantities of decomposition products. On the other hand, the reaction does not go rapidly to completion at 20°C. and time periods of from 5 to 7 hours are needed for substantial completion of the oxidation procedure. Longer periods of time will, of course, be necessary if lower reaction temperatures are employed.

An alternative oxidizing agent is the combination of N-chlorosuccinimide and dimethylsulfide, used in conjunction with a tertiary amine such as triethylamine.

Compounds according to Formula V wherein R is $OCH_3$, $R_6$ and $R_7$ are =O, and $R^2$ is CHO can be prepared by low temperature oxidation of the corresponding compound wherein $R^2$ is $CH_3$ by the procedure of Jovanovics et al., U.S. patent 3,899,493.

The starting materials useful to prepare the 4-oxo compounds of this invention — those materials represented by Formula V wherein R is $OCH_3$, $R_6$ is OH and $R_7$ is H— are prepared by hydrolysis of the corresponding C—4 acetates. The compounds according to Formula V in which $R^2$ is $CH_3$ and $R_6$ is acetoxy and $R_7$ is H are known compounds; i.e., vinblastine is disclosed in U.S. Patent 3,097,137, leurosidine in U.S. Patent 3,205,220, leurosine, in U.S. Patent 3,370,057 and deoxy VLB "A" and "B" in *Tetrahedron Letters,* 783 (1958). The corresponding compounds in which $R^2$ is CHO are also known and include vincristine, also disclosed in U.S. Patent 3,205,220, leuroformine, Belgian patent 811,110, 4'-deoxyvincristine and 4'-deoxy-1-formylleurosidine in the copending US-application of Gerald L. Thompson, Serial No. 760,595, filed 1—19—77. The remaining compound, 1-desmethyl-1-formylleurosidine is prepared by the low temperature oxidation of leurosidine with chromic oxide according to the procedure of U.S. patent 3,899,493. The corresponding 4-desacetyl compounds are prepared from

the above vinca alkaloids by hydrolysis either under acidic or basic conditions. Hargrove, *Lloydia, 27,* 340, (1964) first prepared 4-desacetyl vinblastine by the acidic hydrolysis of vinblastine using large volumes of absolute methanol saturated at 0°C. with anhydrous hydrogen chloride. A contact time of about 6 hours was used. Acidic hydrolysis also seems to be the procedure of choice for preparing 4-desacetyl deoxy VLB "B" and "A". However, it has been found that an alkaline hydrolysis procedure is preferred for the preparation of 4-desacetyl derivatives of both leurosine and vinblastine. According to this procedure, sodium carbonate in refluxing methanol is employed as the hydrolytic medium.

The alkaloidal bases of Formula V of this invention wherein $R^6$ is H, $R^7$ is $OR_1'$, R' is H, R is $OCH_3$ and $R^2$ is $CH_3$ or CHO are prepared by reducing a compound of the formula

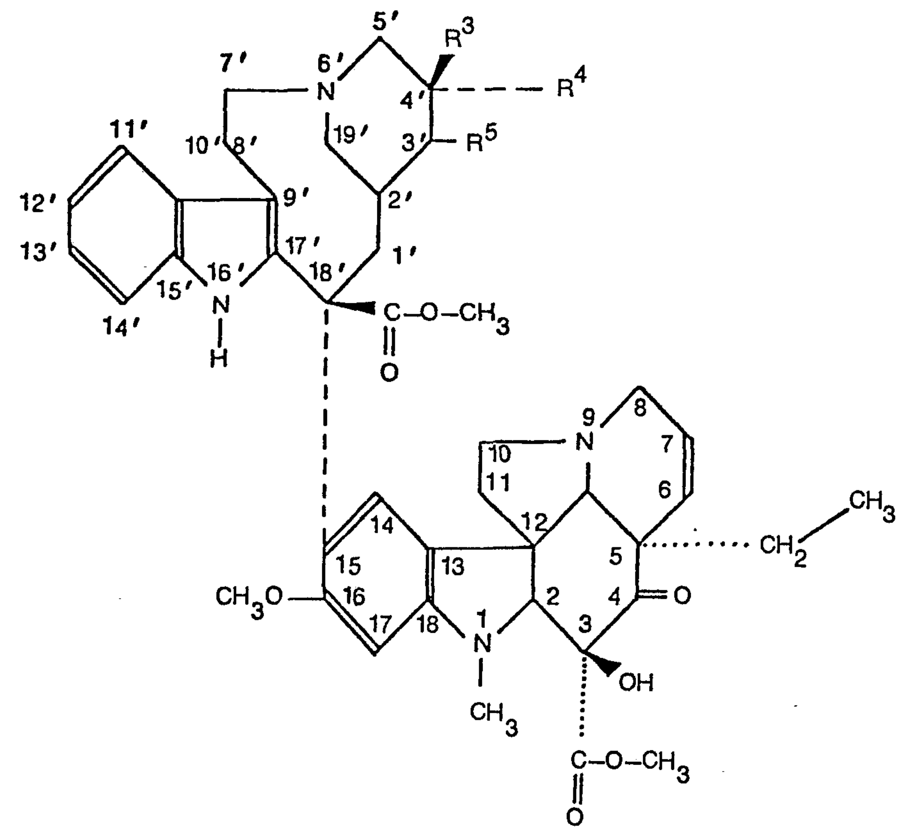

III

wherein $R^3$, $R^4$ and $R^5$ have the same meaning as hereinabove, with LiAlH(t-butyloxy)$_3$ in dry THF (tetrahydrofuran). An excess of reducing agent is customarily employed in this reaction. Other non-reactive anhydrous solvents such as diethylether can be employed in place of THF.

Compounds according to Formula V in which $R^2$ is CHO, R is $OCH_3$, and $R^7$ is $OR^1$ are prepared by low temperature chromic acid-acetic acid oxidation of the corresponding compound in which $R^2$ is methyl. The reaction conditions employed are in general those of Jovanovics *(Loc. cit).* Compounds in which R is NH—$NH_2$, $R^2$ is CHO or $CH_3$, and $R^7$ is $OR^1$ are prepared by reacting hydrazine hydrate with the corresponding compound in which R is methoxy, prepared by the procedures indicated above. Compounds in which $R^2$ is methyl or formyl and R is methoxy and $R^1$ is acetyl are prepared by acetylation of the corresponding compound in which $R^1$ is H. The following compounds illustrate the scope of this invention:

4-desacetoxy-4α-acetoxyleurosine
4-desacetoxy-4α-acetoxyvinblastine
4-desacetoxy-4α-acetoxyleuroformine
4'-deoxy-4-desacetoxy-4α-acetoxyleurosidine
4'-deoxy-4-desacetoxy-4α-hydroxyvinblastine
4'-deoxy-4-desacetoxy-4α-hydroxy-1-formylleurosidine
4-desacetoxy-4α-acetoxyvincristine
4-desacetoxy-4α-hydroxyleurosine
4'-deoxy-4-desacetoxy-4α-hydroxyvincristine

This invention is further illustrated by the following specific examples.

## Example 1

4-Desacetoxy-4-oxovinblastine

A reaction mixture was prepared by adding 3.0739 g. of 4-desacetylvinblastine and 5.8929 g. of dicyclohexylcarbodiimide to 20 ml. of DMSO. 1.2185 Grams of ortho phosphoric acid (anhydrous) dissolved in 5 ml. DMSO were added. The reaction flask was stoppered and swirled until solution was complete. The reaction mixture was maintained at ambient temperature (18—22°C.) for 5 hours, and was then poured into a mixture of ice and 1% aqueous sulfuric acid. The precipitated dicyclohexylurea was removed by filtration and the filter cake washed with 1% aqueous sulfuric acid. The aqueous layer was made basic with 14 N aqueous ammonium hydroxide. This aqueous alkaline mixture was extracted several times with methylene dichloride. The methylene dichloride extracts were combined and dried and the solvent removed therefrom by evaporation in vacuo leaving a residue containing 4-desacetoxy-4-oxovinblastine (2.6534 g.). The residue was dissolved in 1% aqueous sulfuric acid, the resulting solution filtered, and the filtrate made basic with 14 N $NH_4OH$ as before. Extraction with $CH_2Cl_2$ followed by drying and removal of the solvent yielded a residue. Thin-layer chromatography of an aliquot over silica gel using a 1:1 ethyl acetate/ethanol solvent mixture showed the residue to consist of essentially one-spot material. The residue was combined with 298 mg. of a similar residue from a chromatographic separation procedure in a previous run, and the combined material dissolved in 400 ml. of a 0.1 M citric acid buffer, (pH = 2.35) and subjected to a gradient pH extraction procedure. The acidic solution was extracted with 400 ml. of benzene and the pH adjusted to 3.0. The solution was again extracted with 400 ml. of benzene, the pH raised again, etc. The following chart gives the pH of the solution in column 1 and in column 2, the weight of material in the fraction after removal of the benzene. Each fraction was analyzed by thin-layer chromatography as before for the presence of 4-desacetoxy-4-oxovinblastine.

Table 1

| pH of aqueous layer | weight in mg. of extracted product | |
|---|---|---|
| 2.35 | 205.8 | (discarded) |
| 3.0 | 167.1 | |
| 3.5 | 270.6 | |
| 4.0 | 671.4 | (combined) |
| 4.5 | 740.1 | (combined) |
| 5.0 | 257.7 | |
| 5.5 | 123.0 | |
| 6.0 | 66.7 | |
| 6.5 | 26.7 | (discarded) |
| 7.0 | 23.8 | (discarded) |
| 7.5 | 12.8 | (discarded) |
| 8.0 | 22.7 | (discarded) |
| 9.0 | 17.8 | (discarded) |

Fractions taken at pH 3.0—5.0 were combined with a filtrate from a crystallization of 4-desacetoxy-4-oxovinblastine obtained previously by chromatography, and the combined fractions dissolved in 400 ml. of 0.1 m citric acid buffer as before. A second gradient pH extraction procedure was carried out as above except that, in some instances, more than one extraction with a 400 ml. portion of benzene was carried out. Table 2 gives the results of this extraction procedure. Column 1 gives the pH of the aqueous layer, Column 2 the number of extractions with 400 ml. portions of benzene, and Column 3, the weight in milligrams of extracted product after evaporation of the solvent.

Table 2

| pH of aqueous layer | no. of extractions | weight in mg. of extracted product |
|---|---|---|
| 2.35 | 3 | 252.4 (discarded) |
| 2.75 | 2 | 97.1 |
| 3.15 | 1 | 114.2 |
| 3.55 | 1 | 276.1 |
| 4.00 | 3 | 1154.2 |
| 4.5 | 1 | 213.2 |
| 5.0 | 1 | 89.7 |
| 5.5 | 1 | 32.2 |
| 6.0 | 1 | 18.7 |
| 8.0 | 1 | 13.5 |

The fraction at pH = 4.0 weighing 1.1542 g. was crystallized from a methanol/water mixture. 854.3 Milligrams of crystalline 4-desacetoxy-4-oxovinblastine were obtained. Infrared and NMR spectra were in accordance with the proposed structure.

Example 2

Alternate Preparation of 4-Desacetoxy-4-oxovinblastine

A solution was prepared containing 2.47 g. of 4-desacetylvinblastine in 37 ml. of toluene and 7.4 ml. of methylene dichloride. 1.29 Grams of N-chlorosuccinimide were suspended in 30 ml. of anhydrous toluene and the suspension stirred under a nitrogen atmosphere with cooling. 1.06 Milliliters (0.9 g.) of dimethylsulfide were added to this suspension in dropwise fashion. The mixture was then stirred for an additional 15 minutes. Next, the solution of the 4-desacetylvinblastine was added in dropwise fashion over a period of 5 minutes at 0°C. to the mixture of N-chlorosuccinimide and dimethylsulfide in toluene. (The following volumes of solvents were used: 15 times weight of starting material for toluene on a weight/volume basis, 3 times weight of starting material on a weight/volume basis of starting material for N-chlorosuccinimide and an excess of dimethylsulfide. After stirring for 5.6 hours at 0°C., 2.02 ml. (1.47 g.) of triethylamine were added and the reaction mixture was stirred for an additional one-half hour at room temperature and then stored overnight in the refrigerator. Thin-layer chromatography indicated that all of the starting material, 4-desacetylvinblastine, had been consumed, and that the reaction mixture consisted essentially of single spot material. The reaction mixture was diluted with ether plus methylene dichloride and the resulting organic layer washed three times with water and then dried. Removal of the solvents in vacuo yielded a yellow solid comprising 4-desacetoxy-4-oxovinblastine; weight = 2.39 g. This material was chromatographed over 240 g. of activity I silica. The chromatogram was developed with 400 ml. portions of 1:1 methylene dichloride/ethyl acetate containing 9, 13.5, 20, 30, and 45 percent added methanol. After 700 ml. of eluent had been collected, 20 ml. fractions were then taken. Fractions 21—45 yielded, when combined and the solvents evaporated, 1.28 g. of a tan solid, 4-desacetoxy-4-oxovinblastine.

4-Oxo-4-desacetoxyleurosidine, 4-oxo-4-desacetoxyvincristine, 4-oxo-4-desacetoxy-1-desmethyl-1-formylleurosidine, 4-oxo-4-desacetoxyleurosine, 4-oxo-4-desacetoxy-1-leuroformine, 4-oxo-4-desacetoxy-4′-deoxy VLB, 4-oxo-4-desacetoxy-4′-deoxyleurosidine and the corresponding 1-formyl

derivatives are prepared in similar fashion by oxidizing the corresponding 4-desacetyl compound with Moffat's Reagent.

Example 3

Preparation of 4-Desacetoxy-4$\alpha$-hydroxyvinblastine

A solution was prepared from 76.6 mg. of 4-desacetoxy-4-oxovinblastine and 0.90 ml. of anhydrous tetrahydrofuran (THF). All equipment used in forming the solution and carrying out the reaction was oven dried. 76.2 Milligrams of $LiAlH(t\text{-}BuO)_3$ were added to the above solution. The reaction mixture was stirred at room temperature in a sealed flask under nitrogen atmosphere for about 22 hours. Next, a solution of 0.11 g. of ammonium sulfate and 0.17 ml. of water were added. Infusorial earth was next added and the reaction mixture filtered through infusorial earth. The filter cake was washed with THF. The clear colorless filtrate was concentrated *in vacuo* and the resulting residue comprising 4-desacetoxy-4$\alpha$-hydroxyvinblastine formed in the above reaction was dissolved in methylene dichloride. Removal of the methylene dichloride gave 78.7 mg. of a white solid which was shown by TLC to be chiefly one spot material. The residue was chromatographed over 20 g. of Woelm silica gel using 2:1 benzene chloroform containing 6, 9, 13.5, 20, 30, and 45 percent methanol for each 30 ml. portion of eluant. Ten ml. fractions were taken. Fractions 7—12 were combined to give, after evaporation of the solvents, 55.1 mg. of a bright yellow solid shown to be essentially one-spot material on TLC. Recrystallization from ethanol yielded 28.8 mg. of fluffy white crystals. 4-Desacetoxy-4$\alpha$-hydroxyvinblastine thus prepared has the following physical characteristics:

100 MHZ pmr spectrum:

$\delta(CDCl_3)$ 8.23 (bs, 1, indole N—H), 7.43—7.61 (m, 1, $C_{11}$—H), 7.01—7.22 (m, 3, $C_{12'-14'}$—H), 6.59 (s, 1, $C_{14}$—H), 6.05 (s, 1, $C_{17}$—H), 5.66—5.77 (m, 2, $C_{6,7}$—H), 4.02 (d, J=3, 1, $C_4$—H), 3.88 (s, 3, $C_{15}$—$OCH_3$), 3.75 (s, 3, $C_3$—$CO_2CH_3$), 3.73 (bs, 1, $C_2$—H), 3.59 (s, 3, $C_{18'}$—$CO_2CH_3$), 2.85 (s, 3, N—$CH_3$), 0.98 and 0.87 (2t, J=7, 6, $C_{21,22'}$—H).

Infra-red spectrum:

$\nu(CHCl_3)$ = 3465, 3005, 1724, 1616, 1500, 1458, 1432 $cm^{-1}$.

Ultra-violet spectrum:

$\lambda$(EtOH) = 214 ($\varepsilon$4.46 × $10^4$), 288, 296 nm.

Mass spectrum:

m/e 768 ($M^+$), 737, 709, 651, 543, 154.

Example 4

Preparation of 4-Desacetoxy-4$\alpha$-hydroxyvincristine

A solution was prepared from 76.8 mg. of 4-desacetoxy-4$\alpha$-hydroxyvinblastine and 10 ml. of acetone. 43.5 $\mu$l. of an acidic solution prepared from 19.9 ml of water and 2.5 ml. of 18 M sulfuric acid were added. A fine precipitate formed immediately. This acidic mixture was cooled to about −60°C. and an oxidizing solution containing 90 mg. of chromium trioxide in 1 ml. of glacial acetic acid and 0.1 ml. of water was added thereto in dropwise fashion over a 4-minute period. The reaction mixture was stirred in the range −60 to −50°C. for twenty minutes and then cooled to −70°C. Two milliliters of 14 N aqueous ammonium hydroxide were added and the resulting mixture poured into 50 ml. of an ice-water mixture. The aqueous layer was extracted three times with chloroform, and the chloroform extracts combined. The combined extracts were washed with dilute sodium bisulfite and then dried. Evaporation of the solvent yielded 61.6 mg. of a light yellow solid residue. The residue was chromatographed over 20 g. of Woelm activity I silica gel. The chromatogram was developed with 30 ml. portions of 1:1 methylenedichloride/ethyl acetate containing 4, 6, 9, 13.5, 20, and 30 percent methanol. Ten milliliter fractions were taken. Fractions 15 to 20 were combined to give 34.8 mg. of a white solid consisting of 4-desacetoxy-4$\alpha$-hydroxyvincristine formed in the above reaction.

The residue was dissolved in anhydrous ethanol and 124 $\mu$l. of 2 percent v/v aqueous sulfuric acid in anhydrous ethanol was added, thus forming the sulfate salt of 4-desacetoxy-4$\alpha$-hydroxyvinblastine. The free base had the following physical characteristics: Mass spectrum: m/e 782 ($M^+$), 751, 355, 154.

Example 5

Preparation of 4-Desacetoxy-4$\alpha$-hydroxyvinblastine C—3 carboxhydrazide

A reaction mixture was prepared from 76.8 mg. of 4-desacetoxy-4$\alpha$-hydroxyvinblastine, 6 ml. of anhydrous hydrazine (97 percent) and 9 ml. of anhydrous methanol. The reaction vessels were oven dried and flushed with nitrogen. The reaction mixture was heated at 53°C. for 24 hours and was then concentrated *in vacuo*. The resulting residue was twice dissolved in methylene dichloride and the resulting methylene dichloride extract concentrated *in vacuo* to yield a white solid residue which showed one major spot on TLC and no spot corresponding to starting material. The white solid was chromatographed over 22 g. of Woelm activity I silica gel. The chromatogram was developed with 30 ml. portions of 2:1 benzene/chloroform containing 4, 6, 9, 13.5, 20, 30, and 45 percent methanol. Ten milliliter fractions were taken. Fractions 15—23 were combined to yield 54.3 mg. of 4-desacetoxy-4$\alpha$-hydroxyvinblastine C—3 carboxhydrazide, having the following physical characteristics:

Mass spectrum: m/e 768 ($M^+$), 737, 709, 651, 154

Infrared spectrum: $\nu(CHCl_3)$ 343), 3050, 1720, 1655, 1615, 1497, 1455, 1428, 1220 $cm^{-1}$.

Example 6

Preparation of 4-EPI-VLB (4-desacetoxy-4$\alpha$-acetoxy VLB)

A reaction mixture containing 440 mg. of 4-desacetoxy-4$\alpha$-hydroxy VLB, 6 ml. of acetic anhydride and 6 ml. of pyridine, was prepared and allowed to remain at ambient temperature for 17 hours. Volatile constituents were removed in vacuo at 30°C. The residual oil comprising 4-epi VLB formed in the above acetylation, was dissolved in methylene chloride. The organic layer was washed with cold water. The aqueous layer was separated and extracted with methylene chloride. This methylene chloride extract was added to the original layer and the combined layers were dried. Evaporation of the solvent yielded a residue of 4-epi-VLB which was purified by chromatography over 20 g. of silica gel (Activity I), using 1:1 methylene chloride/ethyl acetate containing increasing amounts (9, 13.5, 30 and 45 percent) of methanol as the eluting solvent. Fractions shown to contain 4-epi-VLB by TLC were combined and the solvent removed from the combined extracts. The resulting residue was a yellow solid comprising 14-epi-VLB with the following physical characteristics:

100 MHZ pmr spectrum:

$\delta(CDCl_3)$ 8.41 (br s, 1, indole N—H), 7.39—7.58 (m, 1, $C_{11}$—H), 7.0—7.3 (m, 3, $C_{12'-14'}$—H), 6.58 (s, 1, $C_{14}$—H), 6.03 (s, 1, $C_{17}$—H), 5.49—5.82 (m, 2, $C_{6,7}$—H), 4.56 (s, 1, $C_4$—H), 3.79 (s, 3, $C_{15}$—$OCH_3$), 3.75 (s, 3, $C_3$—$CO_2CH_3$), 3.60 (s, 3, $C_{18'}$—$CO_2CH_3$), 2.90 (s, 3, N—$CH_3$), 1.25 and 0.90 (2t, J=7, 6, $C_{21,21'}$—H).

Infra-red spectrum:

$\nu(CHCl_3) = 3450, 3000, 1730, 1225\ cm^{-1}$.

Ultra-violet spectrum:

$\lambda(EtOA) = 214\ (\varepsilon 4.46 \times 10^4), 288, 296$ nm.

Mass spectrum:

m/e 810 ($M^+$), 779, 751, 469, 295, 154.

The sulfate salt was prepared in ethanolic sulfuric acid at pH = 2.

Leurosidine, deoxy VLB "A", deoxy VLB "B" and leurosine can be subjected to the same series of reactions; i.e., mild alkaline hydrolysis to form the 4-desacetyl derivative, oxidation of the 4-hydroxy derivative to the 4-oxo derivative, reduction of the 4-oxo derivative with $LiAlH(t\text{-}BuO)_3$ to yield the corresponding 4-desacetoxy-4$\alpha$-hydroxy derivative. Each of these derivatives can then be oxidized to yield the corresponding vincristine-type derivative (compounds wherein $R^2$ is CHO); then any of the compounds can be reacted with hydrazine to yield the corresponding C—3 carboxhydrazide.

Any of the 4$\alpha$-hydroxy derivatives of this invention except those in which R is NH—$NH_2$ can be acetylated with acetic anhydride in the presence of base to yield a corresponding 4$\alpha$-acetoxy derivative.

The compounds of this invention are mitotic inhibitors in Chinese hamster ovary cells. In particular, 4-desacetoxy-4$\alpha$-hydroxyvinblastine shows a mitotic inhibition roughly corresponding to that seen with either vinblastine sulfate or with 4-desacetylvinblastine, manifesting an activity as low as 0.01 mcg/ml.

The compounds of this invention, as represented by Formula V wherein $R^7$ is $OR^1$, are antitumor agents. They demonstrate this activity against transplanted tumors in mice. For this determination, a protocol was used which involved the administration of the drug by the intraperitoneal route at a given dose level for 7—10 days after innoculation with the tumor or alternatively, on the first, fifth, and ninth days after innoculation.

The following table — Table 3 — give the results of several experiments in which transplanted tumors in mice were treated successfully with a compound of this invention.

In the table, column 1 gives the name of the compound; column 2, the transplanted tumor; column 3, the dose level or dose level range and the number of days the dosage was administered; and column 4, the percent inhibition of tumor growth or percent prolongation of survival time, e.g., B16. (GLS is an abbreviation for Gardner lymphosarcoma; CA755 is an adenocarcinoma; and B16 is a melanoma.).

Table 3

| Compound | Tumor | mg./kg. x Days | Percent Inhibition or Prolongation of Survival Time |
|---|---|---|---|
| 4-Desacetoxy-4α-hydroxy-vinblastine | GLS | 6.0 x 10 | 100 (day 7) toxic |
| | | 3.0 x 10 | 91 (day 7) 78 (day 11) |
| | | 1.5 x 10 | 35 (day 7) 25 (day 11) |
| | B16 | 9.0 x 3 | toxic |
| | | 6.0 x 3 | 90 |
| | | 3.0 x 3 | 65 |
| | 755 | 3.0 x 10 | 38 |
| 4-Desacetoxy-4α-hydroxy-vincristine sulfate | GLS | 6.0 x 10 | 100 (day 7) toxic |
| | | 3.0 x 10 | 100 (day 7) 100 (day 11) |
| | | 1.5 x 10 | 36 (day 7) 29 (day 11) |
| 4-Desacetoxy-4α-hydroxy-vinblastine C—3 carbox-hydrazide | GLS | 6.0 x 10 | toxic |
| | | 3.0 x 10 | 100 (day 7) 100 (day 11) |
| | | 1.5 x 10 | 97 (day 7) 98 (day 11) |
| | B16 | 9.0 x 3 | toxic |
| | | 6.0 x 3 | 121* |
| | | 3.0 x 3 | 67 |

*Indefinite Survivors

In utilizing the novel compounds of this invention as anti-tumor agents, either the parenteral or oral route of administration may be employed. For oral dosage, a suitable quantity of a pharmaceutically-acceptable salt of a base according to Formula II formed with a non-toxic acid, such as the sulfate salt, is mixed with starch or other excipient and the mixture placed in telescoping gelatin capsules each containing from 7.5 to 50 mg. of active ingredients. Similarly, the anti-neoplastically active salt can be mixed with starch, a binder and a lubricant and the mixture compressed into tablets each containing from the 7.5—50 mgs. of salt. The tablets may be scored if lower or divided dosages are to be used. Parenteral administration is preferred however. For this purpose, isotonic solutions are employed containing 1—10 mg./ml. of a salt of an indole-dihydroindole of Formula II such as the sulfate salt. The compounds are administered at the rate of from 0.01 to 1 mg/kg. and preferably from 0.1 to 1 mg./kg. of mammalian body weight once or twice a week or every two weeks depending on both the activity and the toxicity of the drug. An alternative method of arriving at a therapeutic dose is based on body-surface area with a dose in the range 0.1 to 10 mg./meter squared of mammalian body surface every 7 or 14 days being administered.

In utilizing a compound of this invention clinically, the clinical physician would administer the compound initially by the same route and in the same vehicle and against the same types of tumors as are indicated for vincristine or VLB. The dose levels employed would reflect the difference in dose levels found in the treatment of experimental tumors in mice, the dose levels of the compounds of this invention being less than those used with vincristine and VLB. In clinical tests, as with other anti-tumor agents, particular attention would be paid to the effect of the oncolytic compounds of this invention against the ten "signal" tumors set forth at page 266 of "The Design of Clinical Trials in Cancer Therapy" edited by Staquet (Futura Publishing Company, 1973).

## Claims

1. An indole-dihydroindole of the formula

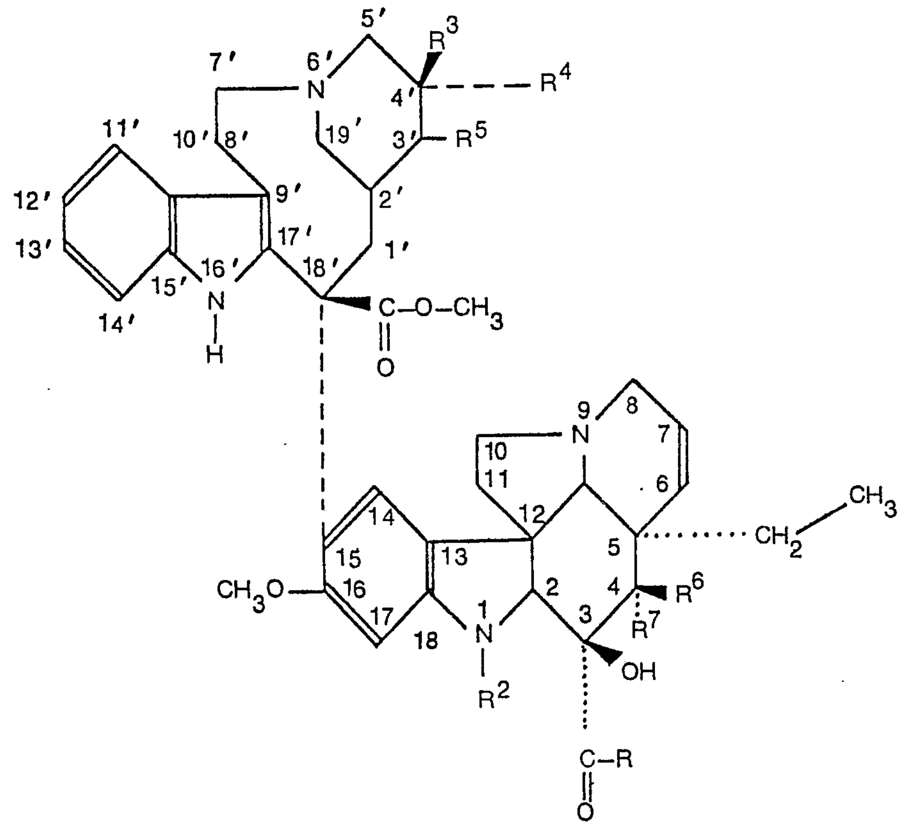

V

wherein:

$R^2$ is $CH_3$ or CHO;

when taken singly, $R^5$ is H and one of $R^3$ and $R^4$ is OH or H and the other is $C_2H_5$ and when $R^4$ and $R^5$ are taken together, they form an $\alpha$-epoxide ring and $R^3$ is $C_{2H5}$,

when taken singly $R^6$ is H and $R^7$ is $OR^1$ and when $R^6$ and $R^7$ are taken together they are =O;

$R^1$ is H or acetyl; and

R is $OCH_3$ or $NH—NH_2$ with the proviso that if R is $—NH—NH_2$, $R^1$ is H; or a pharmaceutically acceptable acid addition salt thereof.

2. 4-Desacetoxy-4$\alpha$-hydroxyvinblastine.

3. 4-Desacetoxy-4-oxo-vinblastine.

4. A process for preparing an indoledihydroindole of the formula

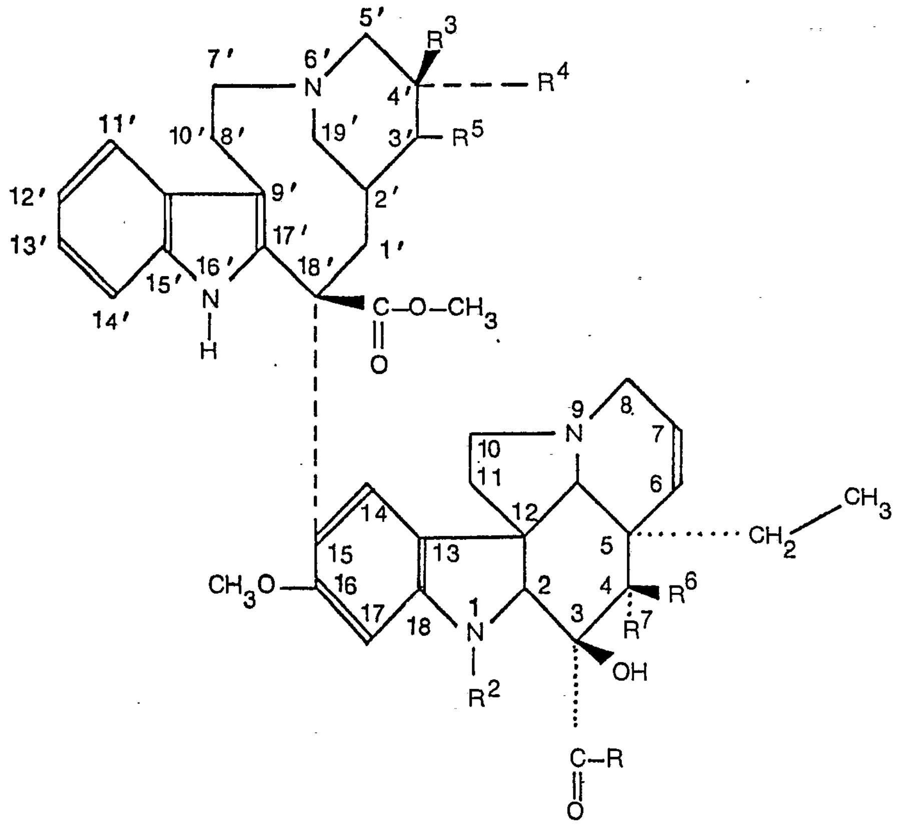

V

wherein:

$R^2$ is $CH_3$ or CHO;

when taken singly, $R^5$ is H and one of $R^3$ and $R^4$ is OH or H and the other is $C_2H_5$ and when $R^4$ and $R^5$ are taken together, they form an $\alpha$-epoxide ring and $R^3$ is $C_2H_5$,

$R^6$ is H;

$R^7$ is $OR^1$ wherein $R^1$ is H;

and R is $OCH_3$

which comprises reacting an indole-dihydroindole of the above formula wherein R, $R^1$, $R^2$, $R^3$, $R^4$ and $R^5$ are as above and wherein $R^6$ and $R^7$ together are =O with a reducing agent in a non-reactive anhydrous solvent and recovering as a free base or a pharmaceutically acceptable salt thereof; and optionally, preceded by reacting an indoledihydroindole of the above formula wherein R, $R^1$, $R^2$, $R^3$, $R^4$ and $R^5$ are as above and wherein $R^6$ is OH and $R^7$ is H with an oxidizing agent in a liquid phase selected from (a) N-chlorosuccinimide and dimethylsulfide with a tertiary amine and (b) dicyclohexyl carbodiimide, o-phosphoric acid and dimethylsulfoxide.

5. A process according to claim 4 for preparing a 4-desacetyl-4$\alpha$-hydroxy indole-dihydroindole of formula V wherein the reducing agent is LiAlH(t-butyloxy)$_3$.

6. A process according to either of claims 4 and 5 for preparing a 4-desacetyl-4$\alpha$-hydroxyindole-dihydrovinblastine of formula V wherein an indole-dihydroindole is reacted with LiAlH(t-butyloxy)$_3$ in anhydrous tetrahydrofuran at room temperature under nitrogen atmosphere with stirring for 22 hours.

7. A process according to any of claims 4 to 6 for preparing 4-desacetoxy-4$\alpha$-hydroxy vinblastine by reacting 4-desacetyl-4-oxo-vinblastine with LiAlH(t-butyloxy)$_3$ in anhydrous tetrahydrofuran at room temperature under nitrogen atmosphere with stirring for 22 hours.

8. A process according to claim 4 for preparing 4-desacetyl-4-oxo-indole-dihydroindole wherein the oxidizing agent is dicyclohexyl carbodiimide, o-phosphoric acid and dimethylsulfoxide.

9. A process according to either of claims 4 and 8 for preparing 4-desacetyl-4-oxovinblastine wherein 4-desacetyl-4$\beta$-hydroxy vinblastine is reacted with dicyclohexylcarbodiimide, o-phosphoric acid and dimethyl sulfoxide at 18° to 22°C. for 5 hours.

10. A pharmaceutical composition which comprises an inert ingredient and as active ingredient an indole-dihydroindole as defined in claims 1 and 2 provided that $R^6$ and $R^7$ are not together on =O group.

11. A compound of general formula (V) as claimed in claims 1 and 2 provided that $R^6$ and $R^7$ are not together on =O group, or a pharmaceutically-acceptable salt thereof, for use as an antimitotic agent.

**Patentansprüche**

1. Indoldihydroindol der Formel

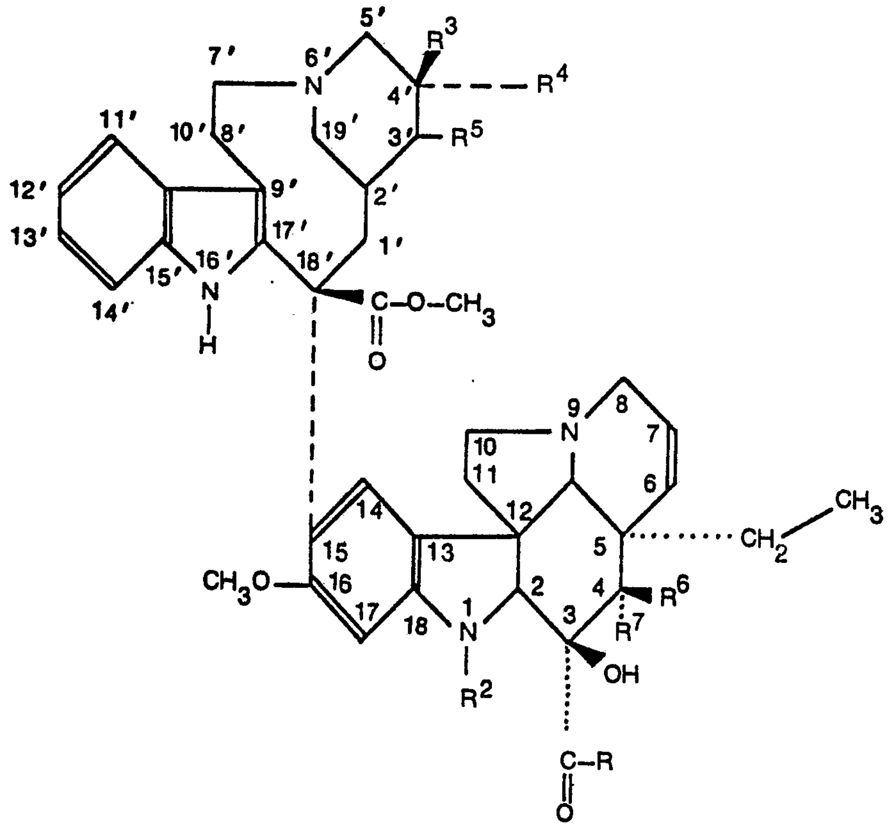

V

worin $R^2$ für $CH_3$ oder CHO steht,

wobei einzeln genommen $R^5$ für H steht und einer der Substituenten $R^3$ oder $R^4$ für OH oder H steht und der andere $C_2H_5$ bedeutet, oder worin $R^4$ und $R^5$ zusammen einen $\alpha$-Epoxidring bilden und $R^3$ für $C_2H_5$ steht,

einzeln genommen $R^6$ für H steht und $R^7$ für $OR^1$ steht, oder $R^6$ und $R^7$ zusammen =O sind,

$R^1$ für H oder Acetyl steht und

R für $OCH_3$ oder NH—$NH_2$ steht,

mit der Maßgabe, daß $R^1$ für H steht, falls R für —NH—$NH_2$ steht,

und die pharmazeutisch unbedenklichen Säureadditionssalze hiervon.

2. 4-Desacetoxy-4$\alpha$-hydroxyvinblastin.

3. 4-Desacetoxy-4-oxovinblastin.

4. Verfahren zur Herstellung eines Indoldihydroindols der Formel

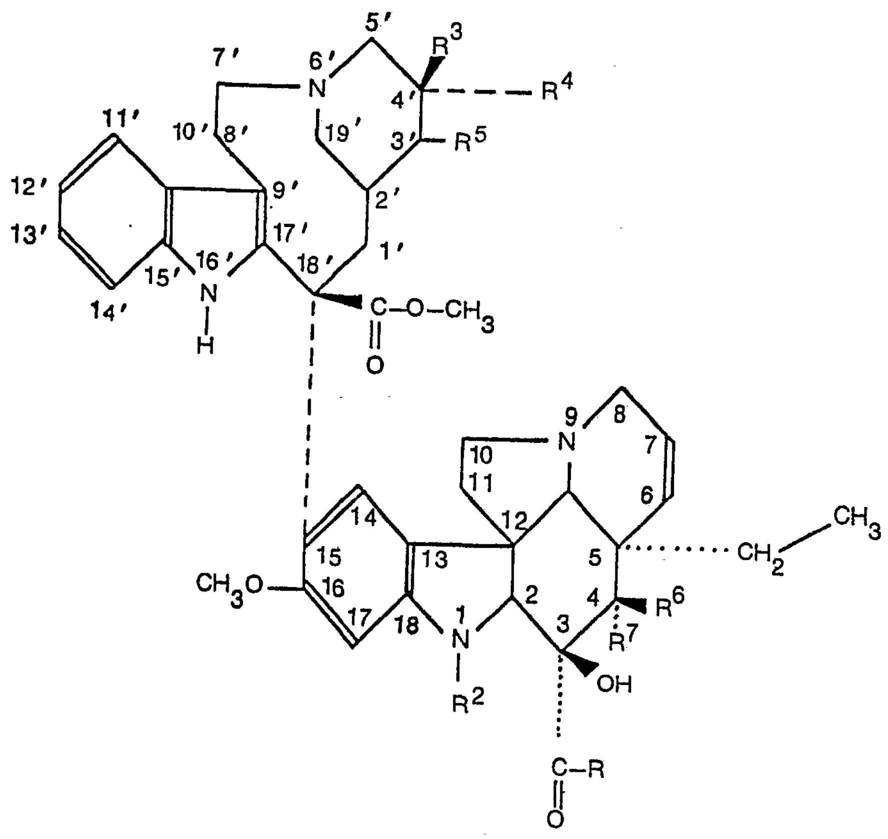

V

worin $R^2$ für $CH_3$ oder CHO steht,

wobei enzeln genommen $R^5$ für H steht und einer der Substituenten $R^3$ oder $R^4$ für OH oder H steht und der andere $C_2H_5$ bedeutet, oder worin $R^4$ und $R^5$ zusammen einen $\alpha$-Epoxidring bilden und $R^3$ für $C_2H_5$ steht,

$R^6$ für H steht,

$R^7$ für $OR^1$ steht, falls $R^1$ für H steht, und

R für $OCH_3$ steht,

dadurch gekennzeichnet, daß man ein Indoldihydroindol der obigen Formel, worin R, $R^1$, $R^2$, $R^3$, $R^4$ und $R^5$ die oben angegebenen Bedeutungen haben und $R^6$ sowie $R^7$ zusammen =O sind, mit einem Reduktionsmittel in einem nicht reaktionsfähigen wasserfreien Lösungsmittel reduziert und die dabei erhaltene Verbindung als freie Base oder pharmazeutisch unbedenkliches Salz gewinnt,

wobei man vorher gegebenenfalls ein Indoldihydroindol der oben angegebenen Formel, worin R, $R^1$, $R^2$, $R^3$, $R^4$ und $R^5$ die obengenannten Bedeutungen haben, $R^6$ für OH steht und $R^7$ für H steht, mit einem Oxidationsmittel in einer flüssigen Phase aus (a) N-Chlorsuccinimid und Dimethylsulfid mit einem tertiären Amin oder (b) Dicyclohexylcarbodiimid, o-Phosphorsäure und Dimethylsulfoxid umsetzt.

5. Verfahren zur Herstellung eines 4-Desacetyl-4$\alpha$-hydroxyindoldihydroindols der Formel V, dadurch gekennzeichnet, daß man als Reduktionsmittel LiAlH(t-Butyloxy)$_3$ verwendet.

6. Verfahren nach einem der Ansprüche 4 oder 5 zur Herstellung eines 4-Desacetyl-4$\alpha$-hydroxyindoldihydrovinblastins der Formel V, dadurch gekennzeichnet, daß man ein Indoldihydroindol mit LiAlH(t-Butyloxy)$_3$ in wasserfreiem Tetrahydrofuran bei Raumtemperatur unter Stickstoffatmosphäre und 22 Stunden langem Rühren umsetzt.

7. Verfahren nach einem der Ansprüche 4 bis 6 zur Herstellung von 4-Desacetoxy-4$\alpha$-hydroxyvinblastin, dadurch gekennzeichnet, daß man 4-Desacetyl-4-oxovinblastin mit LiAlH(t-Butyloxy)$_3$ in wasserfreiem Tetrahydrofuran bei Raumtemperatur unter Stickstoffatmosphäre und 22 Stunden langem Rühren umsetzt.

8. Verfahren nach Anspruch 4 zur Herstellung von 4-Desacetyl-4-oxoindoldihydroindol, dadurch gekennzeichnet, daß man als Oxidationsmittel Dicyclohexylcarbodiimid, o-Phosphorsäure und Dimethylsulfoxid verwendet.

9. Verfahren nach einem der Ansprüche 4 oder 8 zur Herstellung von 4-Desacetyl-4-oxovinblastin, dadurch gekennzeichnet, daß man 4-Desacetyl-4ß-hydroxyvinblastin mit Dicyclohexylcarbodiimid, o-Phosphorsäure und Dimethylsulfoxid bei einer Temperatur von 18 bis 22°C über eine Zeitdauer von 5 Stunden umsetzt.

10. Pharmazeutisches Mittel aus einem inerten Bestandteil und einem Wirkstoff, dadurch gekennzeichnet, daß es als Wirkstoff ein Indoldihydroindol gemäß Anspruch 1 und 2 enthält, mit der Maßgabe, daß $R^6$ und $R^7$ nicht zusammen für =O stehen.

11. Verwendung einer Verbindung der allgemeinen Formel V gemäß Anspruch 1 und 2, mit der Maßgabe, daß $R^6$ und $R^7$ nicht zusammen für =O stehen, oder eines pharmazeutisch unbedenklichen Salzes hiervon als antimitotisches Mittel.

## Revendications

1. Indole-dihydroindole de formule:

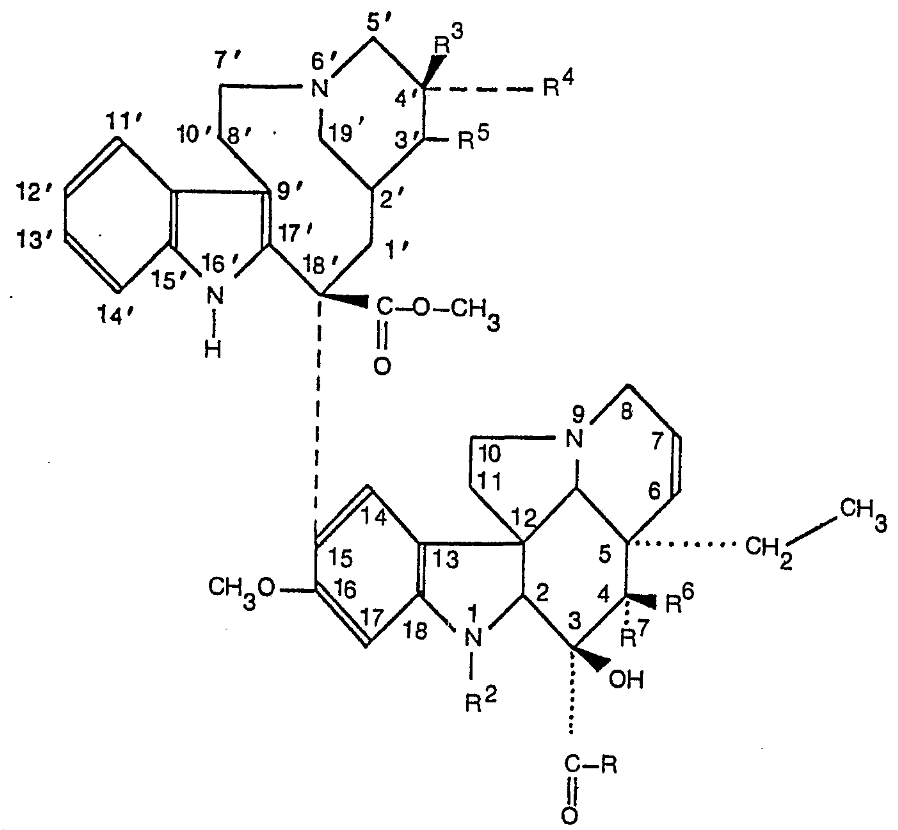

V

dans laquelle:
$R^2$ représente $CH_3$ ou CHO;
pris individuellement, $R^5$ représente H et un des radicaux $R^3$ et $R^4$ représente OH ou H et l'autre représente $C_2H_5$ tandis que, pris ensemble, $R^4$ et $R^5$ forment un noyau $\alpha$-époxyde et $R^3$ représente $C_2H_5$; pris individuellement, $R^6$ représente H et $R^7$ représente $OR^1$ et, pris ensemble, $R^6$ et $R^7$ représentent =O; $R^1$ représente H ou un groupe acétyle; et
R représente $OCH_3$ ou NH—$NH_2$, à condition que, si R représente —NH—$NH_2$, $R^1$ représente H; ou un sel d'addition d'acide pharmaceutiquement acceptable de cet indole-dihydroindole.

2. 4-désacétoxy-4$\alpha$-hydroxyvinblastine.

3. 4-désacétoxy-4-oxo-vinblastine.

4. Procédé de préparation d'un indoledihydroindole de formule:

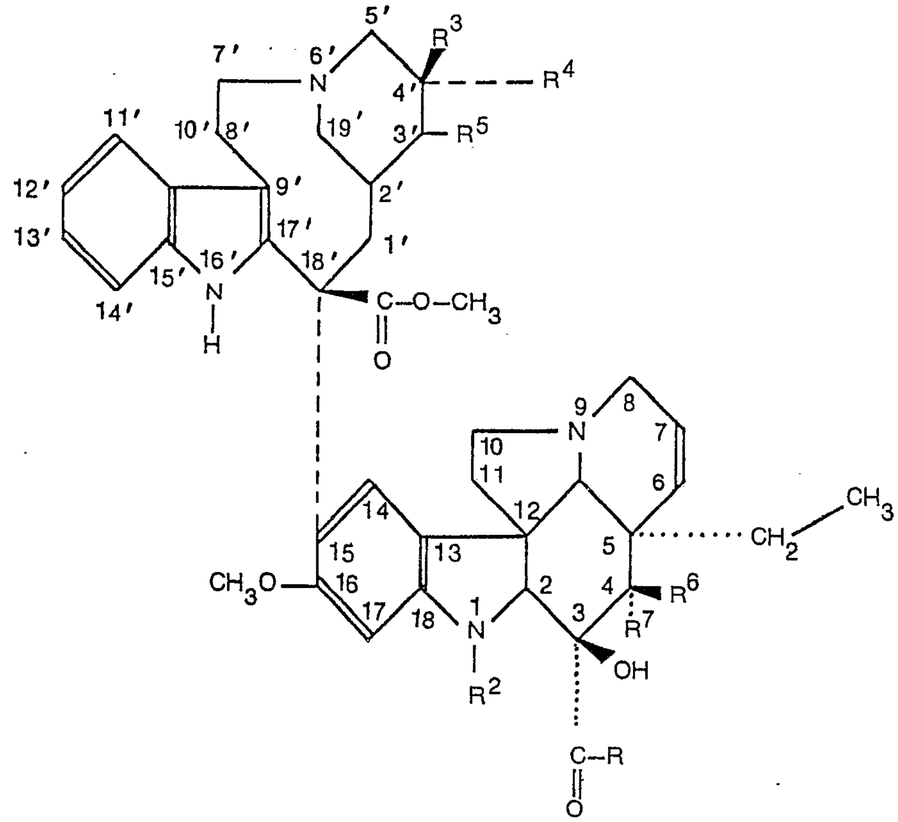

V

dans laquelle:
$R^2$ représente $CH_3$ ou CHO;
pris individuellement, $R^5$ représente H et un des radicaux $R^3$ et $R^4$ représente OH ou H et l'autre représente $C_2H_5$ et, pris ensemble, $R^4$ et $R^5$ forment un noyau $\alpha$-époxyde et $R^3$ représente $C_2H_5$,
$R^6$ représente H;
$R^7$ représente $OR^1$ où $R^1$ représente H;
et R représente $OCH_3$,
caractérisé en ce qu'il consiste à faire réagir un indole-dihydroindole de la formule ci-dessus dans laquelle R, $R^1$, $R^2$, $R^3$, $R^4$ et $R^5$ ont les significations définies ci-dessus, tandis que $R^6$ et $R^7$ ensemble représentent =O, avec un agent réducteur dans un solvant anhydre non réactif et le récupérer sous forme d'une base libre ou d'un sel pharmaceutiquement acceptable,
cette étape étant facultativement précédée d'une réaction d'un indole-dihydroindole de la formule ci-dessus dans laquelle R, $R^1$, $R^2$, $R^3$, $R^4$ et $R^5$ ont les significations définies ci-dessus et dans laquelle $R^6$ représente OH, tandis que $R^7$ représente H, avec un agent oxydant en phase liquide choisi parmi (a) le N-chlorosuccinimide et le sulfure de diméthyle avec une amine tertiaire et (b) le dicyclohexyl-carbodiimide, l'acide o-phosphorique et le diméthylsulfoxyde.

5. Procédé suivant la revendication 4 pour la préparation d'un 4-désacétyl-4$\alpha$-hydroxy-indole-dihydroindole le formule V dans laquelle l'agent réducteur est $LiAlH(t\text{-butyloxy})_3$.

6. Procédé suivant l'une quelconque des revendications 4 et 5 pour la préparation d'une 4-désacétyl-4$\alpha$-hydroxyindole-dihydrovinblastine de formule V, caractérisé en ce qu'on fait réagir un indole-dihydroindole avec $LiAlH(t\text{-butyloxy})_3$ dans du tétrahydrofuranne anhydre à la température ambiante, sous une atmosphère d'azote et tout en agitant pendant 22 heures.

7. Procédé suivant l'une quelconque des revendications 4 à 6 pour la préparation de la 4-désacétoxy-4$\alpha$-hydroxy-vinblastine par réaction de la 4-désacétyl-4-oxo-vinblastine avec $LiAlH(t\text{-butyloxy})_3$ dans du tétrahydrofuranne anhydre, à la température ambiante, sous une atmosphère d'azote et tout en agitant pendant 22 heures.

8. Procédé suivant la revendication 4 pour la préparation du 4-désacétyl-4-oxo-indole-dihydroindole, caractérisé en ce que l'agent oxydant est le dicyclohexyl-carbodiimide, l'acide o-phosphorique et le diméthylsulfoxyde.

9. Procédé suivant l'une quelconque des revendications 4 et 8 pour la préparation de la 4-désacétyl-4-oxo-vinblastine, caractérisé en ce qu'on fait réagir la 4-désacétyl-4$\beta$-hydroxy-vinblastine avec le dicyclohexyl-carbodiimide, l'acide o-phosphorique et le diméthylsulfoxyde à une température de 18 à 22°C pendant 5 heures.

10. Composition pharmaceutique, caractérisée en ce qu'elle comprend un ingrédient inerte et,

comme ingrédient actif, un indole-dihydroindole du type défini dans les revendications 1 et 2, à condition que $R^6$ et $R^7$ ne soient pas ensemble un groupe =O.

11. Composé de formule générale (V) suivant les revendications 1 et 2, à condition que $R^6$ et $R^7$ ne soient pas ensemble un groupe =O, ou un sel pharmaceutiquement acceptable de ce composé, en vue de l'utiliser comme agent antimitotique.